# EUROPÄISCHE PATENTSCHRIFT

(11) **EP 4 152 112 B1**
(45) Veröffentlichungstag und Bekanntmachung des Hinweises auf die Patenterteilung: **19.11.2025**
(21) Anmeldenummer: 21197210.4
(22) Anmeldetag: 16.09.2021
(51) Int. Cl.: G05B 19/402, G05B 19/404, G05B 17/02, G05B 13/04, A61B 6/04

(54) **VERFAHREN ZUM BETRIEB EINER MEDIZINTECHNIKEINRICHTUNG UND MEDIZINTECHNIKEINRICHTUNG**
METHOD FOR OPERATING A MEDICAL DEVICE AND MEDICAL DEVICE
PROCÉDÉ DE FONCTIONNEMENT D'UN DISPOSITIF TECHNIQUE MÉDICAL ET DISPOSITIF TECHNIQUE MÉDICAL

(43) Veröffentlichungstag der Anmeldung: 22.03.2023
(73) Patentinhaber: Siemens Healthineers AG, 91301 Forchheim (DE)
(72) Erfinder: Baumann, Berthold, 95506 Kastl (DE); Krämer, Alexander, 92699 Irchenrieth (DE); Wurzer, Gerhard, 92708 Mantel (DE); Schmidt, Verena, 92681 Erbendorf (DE)
(74) Vertreter: Siemens Healthineers Patent Attorneys

(56) Entgegenhaltungen:
- WO-A1-2020/049214
- GUERRA RODOLFO HABER ET AL: "Digital Twin-Based Optimization for Ultraprecision Motion Systems With Backlash and Friction", IEEE ACCESS, vol. 7, 11 July 2019 (2019-07-11), pages 93462 - 93472, XP011736536, DOI: 10.1109/ACCESS.2019.2928141

## Beschreibung

Die Erfindung betrifft ein Verfahren zum Betrieb einer Medizintechnikeinrichtung, nämlich einer medizinischen Bildgebungseinrichtung, wobei die Medizintechnikeinrichtung wenigstens eine, insbesondere entlang oder um eine Achse, mittels einer Antriebseinrichtung, die durch eine Steuereinrichtung ansteuerbar ist, positionierbare Komponente aufweist, wobei bei Empfang einer Bewegungsanforderung für die Komponente mittels eines Ermittlungsvorgangs Steuerparameter für die Antriebseinrichtung durch die Steuereinrichtung ermittelt werden und die Antriebseinrichtung gemäß der Steuerparameter zur Umsetzung der Bewegungsanforderung angesteuert wird, wobei in den Ermittlungsvorgang wenigstens ein Kompensationsparameter eingeht, der aus einer Mechanikeigenschaftsinformation, die wenigstens eine auf die Positionierungsgenauigkeit der Antriebseinrichtung bezogene Mechanikeigenschaft beschreibt, bestimmt wird. Daneben betrifft die Erfindung eine Medizintechnikeinrichtung.

Viele Medizintechnikeinrichtungen, beispielsweise medizinische Bildgebungseinrichtungen, Patiententische oder medizinische Bildgebungseinrichtungen mit Patiententischen, weisen gegenüber anderen Komponenten und/oder der Umgebung bewegbare Komponenten auf, bei denen eine hohe Positioniergenauigkeit erforderlich ist, damit der Zweck der Medizintechnikeinrichtung in hinreichend guter Qualität erfüllt werden kann. So existieren bei vielen Bildgebungstechniken hohe Positionierungsanforderungen, beispielsweise hinsichtlich einer Aufnahmeanordnung bei der Röntgenbildgebung. Komponenten der Aufnahmeanordnung können beispielsweise an Stativen oder sonstigen Trägern befestigt sein, wo sie mittels einer Antriebseinrichtung, insbesondere bezogen auf unterschiedliche Achsen, in unterschiedliche Stellungen bzw. Positionen verbracht werden können. Ähnlich sind Patiententische meist entlang von drei Achsen verstellbar, beispielsweise in der Höhe (Vertikalachse) bzw. in den beiden Horizontalrichtungen (Längsachse/Querachse). Antriebseinrichtungen können auch dazu ausgebildet sein, Rotationen um bestimmte Achsen herbeizuführen, beispielsweise im Fall eines eine Aufnahmeanordnung für die Röntgenbildgebung tragenden C-Bogens. Auch in anderen Medizintechnikeinrichtungen, beispielsweise bei Therapieeinrichtungen zur Bestrahlung oder dergleichen, ist es bekannt, Komponenten über Antriebseinrichtungen bewegbar zu gestalten.

Zur Ausgestaltung derartiger Antriebseinrichtungen existiert, je nach konkret zu bewegender Komponente und Anwendungsgebiet, eine Vielzahl von Möglichkeiten. Mithin können Antriebseinrichtungen beispielsweise Linearführungen, Schraubspindeln, pneumatische Anteile, Motoren sowie eine Vielzahl weiterer Bestandteile umfassen.

Wie bereits erwähnt, ist bei vielen Medizintechnikeinrichtungen eine hohe, insbesondere definierte, Positioniergenauigkeit der Komponenten erforderlich. Die Positioniergenauigkeit hängt von verschiedenen Mechanikeigenschaften der Antriebseinrichtung ab, beispielsweise von der Steifigkeit des Antriebsstranges, vom vorhandenen Spiel sowie von der Reibung in der Antriebseinrichtung. Dabei kann zudem eine Abhängigkeit dieser Mechanikeigenschaften vom Betriebszustand der Antriebseinrichtung vorliegen, beispielsweise von einer aktuellen Position der Komponente bzw. der Antriebseinrichtung. Eine derartige Positionsabhängigkeit liegt beispielsweise bei Riementrieben vor, bei denen Mechanikeigenschaften, beispielsweise Steifigkeit und/oder Spiel, vom Verhältnis von Lasttrum zu Leertrum abhängen können. Ferner können die Mechanikeigenschaften und somit die Positioniergenauigkeiten, vor allem bei positionsabhängigen Mechanikeigenschaften und/oder niedriger Steifigkeit, wie beispielsweise bei Riementrieben, von dem Gewicht abhängig sein, welches durch die Antriebseinrichtung bewegt werden soll. Dies kann beispielsweise dazu führen, dass bei einem Patiententisch und bei geringem Patientengewicht die Positionsgenauigkeit höher als bei schweren Patienten ist. Dieser Effekt wird durch die Riemenlängenänderung hervorgerufen. Dies gilt letztlich analog für die vorhandene Reibung in einem Riementrieb: Ist diese größer, tritt ebenso eine größere Riemenlängenänderung auf, die in einer geringeren Positioniergenauigkeit resultieren kann.

Dies hat allgemein zur Folge, dass die Antriebseinrichtungen von Medizintechnikeinrichtungen zumeist mit einer sehr großen Steifigkeit und einem sehr geringen Spiel dimensioniert werden, damit die geforderte Positioniergenauigkeit erreicht werden kann. Durch eine äußerst steife Auslegung der Antriebseinrichtungen mit geringem Spiel können die Anforderungen an die Positioniergenauigkeit verbessert erfüllt werden. Da bei sehr steif ausgelegten Antriebseinrichtungen das Gewicht, die Reibung und die Positionsabhängigkeit der Steifigkeit der Antriebseinrichtung nur einen geringen Einfluss auf die Positioniergenauigkeit haben und der Einfluss des Spiels ebenfalls minimiert wurde, können so auch höhere Anforderungen erfüllt werden. Jedoch ist hierbei problematisch, dass ein deutlich höherer Entwicklungsaufwand und deutlich höhere Produktkosten für die Medizintechnikeinrichtung resultieren. Zudem kann es auch bei derartigen Antriebseinrichtungen zu Verschleißerscheinungen kommen, die die Positioniergenauigkeit über die Zeit absinken lassen können.

Guerra Rodolfo Haber et al.: "Digital Twin-Based Optimization for Ultraprecision Motion Systems With Backlash and Friction" offenbart eine modellbasierte Regelungsoptimierung für eine CNC-Maschine.

WO 2020/049 214 A1 offenbart eine modellbasierte Ventilüberwachung.

Der Erfindung liegt daher die Aufgabe zugrunde, eine einfach umsetzbare, kostengünstige und verlässliche Möglichkeit zur Bereitstellung einer hohen Positioniergenauigkeit für Komponenten von Medizintechnikeinrichtungen bereitzustellen.

Zur Lösung dieser Aufgabe ist bei einem Verfahren der eingangs genannten Art erfindungsgemäß vorgesehen, dass zur Überwachung und/oder Ermittlung und/oder Anpassung des wenigstens einen Kompensationsparameters ein Simulationsergebnis eines wenigstens den Betrieb der Antriebseinrichtung abbildenden Simulationsmodells verwendet wird, das mittels wenigstens einem aufgrund einer Ermittlung der Mechanikeigenschaftsinformation zu einem Simulationsmodellparametrierungszeitpunkt bestimmten Simulationsparameter parametriert ist.

Bei der Medizintechnikeinrichtung handelt es sich um eine medizinische Bildgebungseinrichtung, wo die bewegten Komponenten beispielsweise Teile einer Aufnahmeanordnung sein können, bzw. eine medizinische Bildgebungseinrichtung mit einem Patiententisch, bei dem wiederum die Patientenliege die bewegliche Komponente bilden kann. Die Bewegung kann sich beispielsweise auf eine Achse beziehen, beispielsweise linear entlang einer Achse oder aber auf eine Rotation um eine Achse. Konkret kann die Antriebseinrichtung beispielsweise ein Riementrieb sein, welcher beispielsweise für eine Bewegung entlang der Vertikalachse, der Querachse oder der Längsachse für eine Patientenliege eingesetzt werden kann. Hierbei treten durch Spiel und Längenänderung des Riemens sowie dessen Reibung als Mechanikeigenschaften entsprechende Effekte auf, die mittels der Kompensationsparameter bei der Ansteuerung korrigiert und somit zur Erhöhung der Positioniergenauigkeit berücksichtigt werden können, wenn die Mechanikeigenschaften hinreichend genau bekannt sind. Selbstverständlich können jedoch auch andere Antriebseinrichtungen, beispielsweise pneumatische Antriebseinrichtungen, verwendet werden.

Gemäß der vorliegenden Erfindung wird nun vorgeschlagen, bevorzugt mit einer ersten Einstellung der Kompensationsparameter, beispielsweise aufgrund einer Messung der Mechanikeigenschaften zum Erhalt der Mechanikeigenschaftsinformation, auch ein Simulationsmodell wenigstens der Antriebseinrichtung und der zu bewegenden Komponente, bevorzugt der gesamten Medizintechnikeinrichtung oder einer Untereinheit derselben, beispielsweise eines Patiententischs, als einen "digitalen Zwilling" zu erzeugen und zu verwenden. Dabei ist es grundsätzlich denkbar, das Simulationsmodell unmittelbar in den Ermittlungsvorgang für die Steuerungsparameter zu integrieren, bevorzugt ist es jedoch, es als Vergleichsbasis zur Überwachung und/oder Anpassung der Kompensationsparameter heranzuziehen, sodass trotz einer gegebenenfalls weniger steifen Auslegung bzw. einer Auslegung mit mehr Spiel und/oder mit einer stärkeren Abhängigkeit von der Position und/oder anderen Betriebszustandsparametern, eine Aufrechterhaltung einer erforderlichen Positioniergenauigkeit erreicht werden kann. Hierzu werden zu wenigstens einem Zeitpunkt die Mechanikeigenschaften, beschrieben durch die Mechanikeigenschaftsinformation, ermittelt, bevorzugt gemessen, was eine Kalibrierung der Steuereinrichtung hinsichtlich der Kompensationsparameter auch des Simulationsmodells, also des digitalen Zwillings, hinsichtlich der Simulationsparameter ermöglicht. Hierbei kann die Mechanikeigenschaftsinformation selbstverständlich auch bereits so ermittelt werden, dass sie die Kompensationsparameter und/oder die Simulationsparameter wenigstens teilweise bereits umfasst.

Der digitale Zwilling, also das Simulationsmodell, erlaubt also letztlich eine Optimierung der Positioniergenauigkeit insbesondere dahingehend, dass die Kompensation in der Steuereinrichtung durch die Kompensationsparameter ständig überwacht und besonders bevorzugt auch automatisch aktualisiert werden kann. Mithin kann eine geforderte Positioniergenauigkeit idealerweise über die gesamte Lebensdauer der Medizintechnikeinrichtung aufrechterhalten werden und es wird ermöglicht, kostengünstig realisierbare Antriebseinrichtungen bzw. Komponenten derselben, beispielsweise Lager, in Medizintechnikeinrichtungen zu integrieren, ohne dass ein großer Aufwand in deren Weiterentwicklung gesteckt werden muss. Mit anderen Worten liegt ein geringerer Entwicklungsaufwand vor, da durch den digitalen Zwilling letztlich alle relevanten Vorgänge und Eigenschaften, auch bereits im Entwicklungsstadium, abgebildet werden können. Beispielsweise können durch das Simulationsmodell alle relevanten Kräfte, Momente, Abweichungen, Verformungsverhalten, Schwingverhalten, Reibungseffekte und dergleichen bekannt sein. Auf diese Weise kann davon ausgegangen werden, dass bereits ein erster Prototyp nahezu Serienproduktreife aufweist, mithin weniger Entwicklungszyklen erforderlich sind, geringere Kosten anfallen und die Erfahrungen des Simulationsmodells für weitere Entwicklungen herangezogen werden können. Durch die einfachere Auslegung der Antriebseinrichtungen kann auch der Materialeinsatz reduziert werden, sodass die Medizintechnikeinrichtung weniger Masse aufweist. Dies erleichtert die Montage in der Fertigung bzw. am Betriebsort. Allgemein kann gesagt werden, dass eine weniger steife, mithin weiche Antriebseinrichtung, beispielsweise ein weicher Antriebsstrang, gewählt werden kann, da Weichheiten in der Antriebseinrichtung durch die Steuerung berücksichtigt werden, insbesondere auch deren Veränderung über die Zeit überwacht wird und gegebenenfalls eine Anpassung vorgenommen wird.

Selbstverständlich kann im Rahmen der vorliegenden Erfindung jedoch auch mit bereits auf eine hochgenaue Positioniergenauigkeit ausgelegten Antriebseinrichtungen gearbeitet werden und bei diesen ebenso eine höhere Positioniergenauigkeit, insbesondere über die gesamte Lebensdauer, bereitgestellt werden, sodass insbesondere auch Grenzen der Technik, die bei stetiger Verbesserung der Positioniergenauigkeit von Antriebseinrichtungen erreicht werden können, überschritten werden können.

Durch den hier beschriebenen Einsatz können auch mehrere Antriebseinrichtungen in einer Medizintechnikeinrichtung abgedeckt werden. So kann beispielsweise vorgesehen sein, dass die Medizintechnikeinrichtung mehrere Antriebseinrichtungen mit zugeordneten Mechanikeigenschaften, Ermittlungsvorgängen in der Steuereinrichtung und Kompensationsparametern umfasst, wobei für jede Antriebseinrichtung ein Simulationsmodell verwendet wird oder, vorzugsweise, ein Simulationsmodell, insbesondere der ganzen Medizintechnikeinrichtung, für alle Antriebseinrichtungen verwendet wird. In diesem Kontext ist es also besonders bevorzugt, wenn das Simulationsmodell als digitaler Zwilling die gesamte Medizintechnikeinrichtung, beispielsweise einen Patiententisch, letztlich vollständig abbildet und somit insbesondere zur Überwachung und Nachführung aller Kompensationsparameter der verschiedenen Antriebseinrichtungen verwendet werden kann.

Als Mechanikeigenschaft kann eine Reibung und/oder ein Spiel und/oder eine Steifigkeit der Antriebseinrichtung verwendet werden. Selbstverständlich sind grundsätzlich auch andere Mechanikeigenschaften denkbar, die Relevanz für die Positioniergenauigkeit aufweisen und die bei der Ansteuerung entsprechend zu berücksichtigen sind.

Wenigstens eine Mechanikeigenschaftsinformation kann in Abhängigkeit von wenigstens einer den Betriebszustand der Antriebseinrichtung betreffenden Betriebszustandsinformation ermittelt werden. Wie bereits erwähnt, können Mechanikeigenschaften sich in unterschiedlichen Betriebszuständen unterscheiden, beispielsweise hinsichtlich der aktuellen Position der Komponente bzw. der Antriebseinrichtung, des zu bewegenden Gewichts, der Temperatur und dergleichen. Konkret kann beispielsweise vorgesehen sein, dass als Betriebszustandsinformation eine aktuelle Position der Komponente und/oder eine zu bewegende Last und/oder eine Temperatur der Antriebseinrichtung und/oder eine im Rahmen der Steuerung zu verwendende Beschleunigung verwendet wird. Diese Betriebszustandsinformationen können selbstverständlich auch in den Ermittlungsvorgang und/oder die Simulation eingehen, wobei nicht nur Kompensationsparameter und/oder Simulationsparameter gegebenenfalls abhängig von einer Betriebszustandsinformation, beispielsweise als Kennlinie bzw. Kennfeld, bereitgestellt werden können, sondern auch andere Einflüsse, insbesondere in der Simulation, die von diesen Betriebszustandsinformationen ausgeht, berücksichtigt werden können.

Zweckmäßigerweise wird als ein erster Simulationsmodellparametrierungszeitpunkt eine Beendigung der Herstellung verwendet, wobei dann auch eine Kalibrierung der Steuereinrichtung bezüglich der Kompensationsparameter erfolgt. Die Beendigung der Herstellung der Medizintechnikeinrichtung bzw. der Zeitpunkt nach deren Installation/Montage, vor der ersten Inbetriebnahme, stellen einen zweckmäßigen Moment dar, die Mechanikeigenschaftsinformation zu ermitteln und hinsichtlich hochqualitativer und verlässlicher Startwerte für die Kompensationsparameter der Steuerung und die Simulationsparameter des Simulationsmodells auszuwerten, sodass zu Beginn des Betriebs sofort die hohe Positioniergenauigkeit vorliegt und aufgrund der gleichzeitigen Parametrierung des Simulationsmodells auch unmittelbar eine Überwachung beginnen kann.

Erfindungsgemäß ist vorgesehen, dass bei einer Kalibrierung der Steuereinrichtung bezüglich der Kompensationsparameter oder einer Parametrierung des Simulationsmodells bei Inbetriebnahme nach der Installation eine Überprüfungsmessung durchgeführt wird, bei der das Simulationsergebnis mit einem Messergebnis der Überprüfungsmessung verglichen wird, wobei bei Erfüllung eines ersten, eine erste Abweichung zwischen dem Simulationsergebnis und der Überprüfungsmessung anzeigenden Maßnahmenkriteriums eine Neukalibrierung der Steuereinrichtung und eine Neuparametrierung des Modells durchgeführt werden und/oder bei Erfüllung eines zweiten, eine zweite Abweichung zwischen dem Simulationsergebnis und der Überprüfungsmessung anzeigenden Maßnahmenkriteriums eine einen Fehler bei der Installation anzeigende Warnmeldung ausgegeben wird. Das bedeutet, vor der Lieferung einer Medizintechnikeinrichtung werden bereits die für die Positioniergenauigkeit erforderlichen Mechanikeigenschaften, beispielsweise Reibung und Spiel, vermessen, und sowohl der Ermittlungsvorgang in der Steuereinrichtung entsprechend kalibriert sowie der digitale Zwilling, also das Simulationsmodell, damit parametriert. Nach der Installation/Montage der Medizintechnikeinrichtung am Betriebsort wird die Medizintechnikeinrichtung mittels einer Überprüfungsmessung, beispielsweise umfassend ein Testprogramm, getestet und mithin überprüft, ob der digitale Zwilling die realen Bewegungen in der Medizintechnikeinrichtung nachvollzieht. Treten kleinere, erste Abweichungen auf, kann eine neue Ermittlung der Mechanikeigenschaften erfolgen und sowohl die Kompensationsparameter als auch die Simulationsparameter können entsprechend aktualisiert werden, um bei Inbetriebnahme bereits die gewollte Positioniergenauigkeit zu erhalten. Werden jedoch größere, zweite Abweichungen festgestellt, kann auf Montagefehler bzw. allgemeine Installationsfehler geschlossen werden und eine entsprechende Warnung ausgegeben werden, um hier eine Korrektur des Fehlers anzustoßen.

In besonders bevorzugter Ausgestaltung der vorliegenden Erfindung, wie bereits angedeutet, kann vorgesehen sein, dass zur Überwachung und/oder Anpassung der Kompensationsparameter ein während der Ausführung einer Bewegungsanforderung aufgenommenes Messergebnis mit einem die Ausführung der Bewegungsanforderung in dem Simulationsmodell beschreibenden Simulationsergebnis verglichen wird. Werden Betriebszustandsinformation verwendet, werden diese selbstverständlich auch zuvor bestimmt und entsprechend berücksichtigt. Es wird also während der Ausführung einer Bewegungsanforderung, mithin der Nutzung von aufgrund der Kompensationsparameter bestimmten Steuerparametern, etwas gemessen, dass auch aus dem Simulationsmodell durch Simulation hergeleitet werden kann. Treten hierbei Abweichungen auf, ist davon auszugehen, dass die Mechanikeigenschaften in dem Simulationsmodell nicht mit den tatsächlich vorliegenden Mechanikeigenschaften der Medizintechnikeinrichtung übereinstimmen, sodass es zu der Abweichung kommt. So kann auf einfache Art und Weise festgestellt werden, ob noch die die korrekten Mechanikeigenschaften beschreibenden Kompensationsparameter verwendet werden.

Beispielsweise kann in einer bevorzugten Ausführungsform ein Verlauf eines Motorstroms und/oder eines Motordrehmoments eines Motors der Antriebseinrichtung vermessen und simuliert werden. Dies hat den zusätzlichen, besonderen Vorteil, dass aus einem solchen Verlauf des Motorstroms und/oder des Drehmoments eines Motors auch unmittelbar Rückschlüsse über die Mechanikeigenschaften gezogen werden können, wie im Folgenden noch genauer erläutert werden wird. Ferner sind eine Vielzahl eingesetzter Motorcontroller, wie sie kommerziell erhältlich sind, bereits ausgebildet, über eine definierte Schnittstelle entsprechende Messergebnisse für den Motorstrom und/oder das Drehmoment auszugeben. Diese bislang häufig nicht genutzten Optionen werden im Rahmen der vorliegenden Erfindung mithin genutzt, um das Bestehen der gewollten Positioniergenauigkeit ständig zu überwachen und gegebenenfalls eine Aktualisierung zu deren Aufrechterhaltung herbeizuführen.

Konkret kann in diesem Kontext vorgesehen sein, dass bei einer ein Aktualisierungskriterium erfüllenden Abweichung zwischen dem Messergebnis und dem Simulationsergebnis durch neue Ermittlung der Mechanikeigenschaftsinformation die Kompensationsparameter und die Simulationsparameter zur Korrektur der Abweichung angepasst werden. Liegen beispielsweise Abweichungen vor, die wenigstens einen Schwellwert übersteigen, kann daraus geschlossen werden, dass mit den aktuellen Kompensationsparametern die gewollte Positioniergenauigkeit nicht aufrechterhalten werden kann, wobei mithin der wenigstens eine in dem Aktualisierungskriterium verwendbare Schwellwert in Abhängigkeit einer gewollten, aufrechtzuerhaltenden Positioniergenauigkeit gewählt werden kann.

In diesem Zusammenhang kann mit besonderem Vorteil vorgesehen sein, dass die Mechanikeigenschaftsinformation unter Verwendung des Messergebnisses neu ermittelt wird. Mithin kann die vorzunehmende Messung bzw. Simulation gezielt bereits so gewählt werden, dass sich die Mechanikinformation hieraus ohne eine weitere notwendige Messung oder dergleichen ermitteln lässt. Ein vorteilhaftes Beispiel hierfür sind der bereits erwähnte Motorstrom bzw. das Motordrehmoment. Beim Verfahren der Komponente über einen bestimmten Verfahrweg folgen das Motordrehmoment sowie der Motorstrom üblicherweise einem typischen Verlauf. Nach einem bestimmten Zeitraum, der das Spiel der Antriebseinrichtung beschreibt, folgt zunächst ein Anfahrtspeak in einem Anfahrtszeitbereich, dem dann ein relativ konstanter Verlauf während einer Konstantfahrt, nämlich einer Bewegung der Komponente mit einer bestimmten Geschwindigkeit, folgt. Im Bereich dieser Konstantfahrt gibt der dann vorliegende Wert des Motorstroms bzw. Motordrehmoments, beispielsweise dessen Mittelwert, ein Maß für die vorliegende Reibung, insbesondere den Reibwert. Aus diesem Zusammenhängen kann abgeleitet werden, wie entsprechende Kompensationsparameter bezüglich der Mechanikeigenschaften Spiel und Reibung zu aktualisieren sind.

Liegt eine Abhängigkeit von einer Betriebszustandsinformation vor, beispielsweise der Position, der zu bewegenden Last oder der Temperatur, ist es zweckmäßig, auch diese Abhängigkeiten entsprechend zu aktualisieren. Mithin sieht eine zweckmäßige Weiterbildung in diesem Zusammenhang vor, dass, insbesondere bei von einer Betriebszustandsinformation abhängig ermittelter Mechanikeigenschaftsinformation, die Neuermittlung aus mehreren, über einen Zeitraum, insbesondere aber in unterschiedlichen Betriebszuständen, aufgenommenen Messergebnissen erfolgt. Mithin werden über die Zeit hinreichend viele "Stichproben" gesammelt, um die Kompensationsparameter vollständig, also auch hinsichtlich ihrer Betriebszustandsabhängigkeit, aktualisieren zu können, wobei bei einer solchen Vorgehensweise zweckmäßigerweise das Aktualisierungskriterium etwas empfindlicher gestaltet werden kann, beispielsweise bereits vor einem Erreichen bzw. Überschreiten der gewollten Positioniergenauigkeit.

Dabei sei angemerkt, dass neben der bevorzugten Ermittlung der Mechanikeigenschaftsinformation aus wenigstens einem des wenigstens einen Messergebnisses es auch denkbar ist, dass die Neuermittlung wenigstens teilweise durch einen dedizierten Messvorgang und/oder durch ein Optimierungsverfahren mittels des Simulationsmodells, in dem das Simulationsergebnis durch Anpassung der Simulationsparameter auf das Messergebnis angepasst wird, ermittelt wird. Im letzten genannten Ansatz wird letztlich versucht, die Realität abzubilden, indem eine Optimierung bezüglich der Messeigenschaften, die das Simulationsmodell parametrieren, durchgeführt wird, woraus dann folglich Messeigenschaften folgen, für die die Realität entsprechend abgebildet wird. in einem solchen Fall ist vorteilhafterweise keine dedizierte Messung erforderlich.

In besonders vorteilhafter Ausgestaltung kann vorgesehen sein, dass der Vergleich für jede Bewegungsanforderung durchgeführt wird. Das bedeutet in anderen Worten, dass der digitale Zwilling, also das Simulationsmodell, jede Bewegung, die in der realen Medizintechnikeinrichtung stattfindet, ebenfalls durchführt, sodass Abweichungen, auch deren Entwicklung über die Zeit, zeitnah und frühzeitig festgestellt werden können, um beispielsweise eine Aktualisierung herbeizuführen. Nachdem er sich bei dem Simulationsmodell um ein Rechenmodell handelt, ist dies problemlos möglich, insbesondere zumindest im Wesentlichen in Echtzeit.

In einer bevorzugten Weiterbildung der vorliegenden Erfindung kann vorgesehen sein, dass jeder Vergleich und/oder jede Anpassung protokolliert werden, wobei durch Auswertung der Protokolldaten wenigstens eine Verschleißinformation und/oder eine die Notwendigkeit einer Wartung angebende Wartungsinformation ermittelt werden. Das bedeutet, die vorhandene Veränderung der Mechanikeigenschaften, beispielsweise des Spiels und/oder der Reibung, kann über die Zeit mitverfolgt werden, um weitere Schlussfolgerungen zu ziehen, beispielsweise hinsichtlich der Notwendigkeit einer Wartung, hinsichtlich des Verschleißes und dergleichen. Konkret kann beispielsweise vorgesehen sein, dass zur Ermittlung der Wartungsinformation ein Vergleich wenigstens einer aktuellen Mechanikeigenschaft mit einer ursprünglichen, zum ersten Simulationsmodellparametrierungszeitpunkt ermittelten Mechanikeigenschaft und/oder eine Auswertung eines Verlaufs einer Mechanikeigenschaft erfolgt. Beispielsweise kann ein Vergleich mit einem Schwellwert erfolgen, wobei beispielsweise ein aktuelles Spiel und/oder ein aktueller Reibwert mit den Ursprungswerten, insbesondere bei Inbetriebnahme des Systems, verglichen werden können und die Abweichung beurteilt werden kann. Möglich ist es in diesem Zusammenhang beispielsweise, dass als Wartungsinformation ein vorgeschlagener Wartungstermin ausgegeben wird.

Dabei ist es zweckmäßig, wenn die Protokollierung gemeinsam mit einer zugeordneten Betriebszustandsinformation erfolgt, nachdem dies eine verbesserte Auswertung der Protokolldaten ermöglicht, auch falls die Mechanikeigenschaften nicht betriebszustandsabhängig betrachtet werden sollten. Beispielsweise kann dann auf bestimmte Einflüsse gefolgert werden.

Besonders zweckmäßig ist es, wenn Protokolldaten mehrerer Medizintechnikeinrichtungen gemeinsam ausgewertet werden. Auf diese Weise lässt sich Intervariabilität innerhalb einer größeren Menge insbesondere zumindest bezüglich der Antriebseinrichtung baugleicher Medizintechnikeinrichtungen erkennen. So können beispielsweise Probleme, die nur bei einer bestimmten Charge auftreten, identifiziert werden und entsprechende Maßnahmen in die Wege geleitet werden. Auch Beschädigungen oder dergleichen lassen sich, zusätzlich zur Wartungsinformation, die sich auf eine bestimmte Medizintechnikeinrichtung bezieht, über mehrere Medizintechnikeinrichtungen und deren Verhalten über die Zeit hinweg leichter identifizieren.

Zweckmäßigerweise kann auch vorgesehen sein, dass die Protokolldaten, insbesondere mehrerer Medizintechnikeinrichtungen, zur Ermittlung einer bei der Entwicklung neuer Antriebseinrichtungen verwendbaren Entwicklungsinformation ausgewertet werden. Hierbei können mit besonderem Vorteil Betriebszustandsinformationen zusätzlich betrachtet werden, um eine allgemeine Auslastung der Medizintechnikeinrichtung zu bestimmen, wobei derartige Betriebszustandsinformation in den Protokolldaten auch alleine berücksichtigt werden können. So ist es beispielsweise denkbar, die vorhandenen Gewichtsbelastungen und/oder Verfahrwege aller Medizintechnikeinrichtungen automatisch zu vergleichen und für die Auslegung von zukünftigen Systemen heranzuziehen.

In diesem Zusammenhang sei zudem angemerkt, dass es besonders zweckmäßig ist, wenn das Simulationsmodell auf einer zur Medizintechnikeinrichtung externen, mit dieser kommunizierenden Servereinrichtung abgespeichert wird, insbesondere in einer Cloud und/oder gemeinsam mit Simulationsmodellen anderer Medizintechnikeinrichtungen. Auf dieser Weise können mithin Daten, beispielsweise bei einem Hersteller, zusammengeführt werden, welcher diese auch, beispielsweise im Sinne einer Massendatenauswertung ("big data"), für sich weiter nutzen kann. Dabei entsteht nicht nur eine hervorragende Datenbasis, beispielsweise hinsichtlich der Früherkennung von Getriebe- und/oder Lagerschäden, sondern auch eine Datenbasis über die Systembelastungen und Verschleißentwicklungen für zukünftige Auslegungen/Dimensionierungen von Komponenten/Antriebseinrichtungen.

Hinsichtlich der Verschleißinformation und/oder der Wartungsinformation ist noch anzumerken, dass in diesem Zusammenhang mit besonderem Vorteil auch ein Vergleich der Mechanikeigenschaften über den Bewegungsweg hinweg, beispielsweise einen Verfahrweg entlang einer Achse, betrachtet werden kann. Ist beispielsweise die Änderung/Abschwächung des Spiels und/oder der Reibung in einer Antriebseinrichtung bei geringem Bewegungsweg um einen bestimmten Faktor größer als Werte, die zuvor ermittelt wurden, kann dies als ein Anzeichen gesehen werden, dass das Getriebe, die Lager und/oder andere Anteile der Antriebseinrichtung beschädigt sind und ausgetauscht werden sollten.

An dieser Stelle sei noch angemerkt, dass die Verfahrensschritte, welche hier genauer beschrieben wurden sind, selbstverständlich automatisiert durch die Steuereinrichtung bzw. eine Steueranordnung, die die Steuereinrichtung umfasst, ausgeführt werden. Es handelt sich mithin um ein computerimplementiertes Verfahren.

Neben dem Verfahren betrifft die vorliegende Erfindung auch eine Medizintechnikanordnung, umfassend eine Steueranordnung und wenigstens eine Medizintechnikeinrichtung, wobei die Medizintechnikeinrichtung wenigstens eine mittels einer Antriebseinrichtung, die durch eine Steuereinrichtung als Teil der Steueranordnung ansteuerbar ist, positionierbare Komponente aufweist, welche sich dadurch auszeichnet, dass die Steueranordnung zur Durchführung des erfindungsgemäßen Verfahrens ausgebildet ist. Sämtliche Ausführungen bezüglich des erfindungsgemäßen Verfahrens lassen sich analog auf die erfindungsgemäße Medizintechnikanordnungen übertragen, sodass auch mit dieser die bereits genannten Vorteile erhalten werden können.

Insbesondere kann bezüglich der Medizintechnikanordnung vorgesehen sein, dass sie mehrere Medizintechnikeinrichtungen und eine Servereinrichtung zum Vorhalten der Simulationsmodelle aller Medizintechnikeinrichtungen aufweist. Auf einer solchen Servereinrichtung können dann auch die Protokolldaten vorgehalten werden und die entsprechenden Auswertungen vorgenommen werden, insbesondere auch medizintechnikeinrichtungsübergreifend.

Weitere Vorteile und Einzelheiten der Erfindung ergeben sich aus den im Folgenden beschriebenen Ausführungsbeispielen sowie anhand der zugehörigen Zeichnungen. Hierbei zeigen schematisch:
- Fig. 1: einen Ablaufplan eines Ausführungsbeispiels des erfindungsgemäßen Verfahrens,
- Fig. 2: mögliche Verläufe eines Motordrehmoments und
- Fig. 3: eine erfindungsgemäße Medizintechnikanordnung

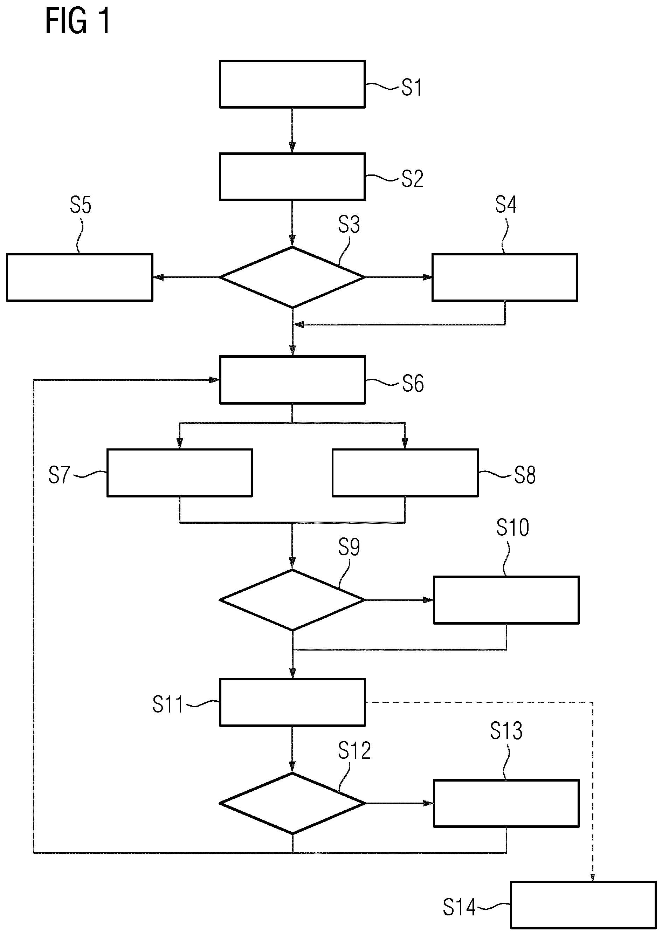

Fig. 1 zeigt ein Ablaufplan eines Ausführungsbeispiels des erfindungsgemäßen Verfahrens. Dabei wird beispielhaft als Medizintechnikeinrichtung ein Patiententisch betrachtet, welcher als Antriebseinrichtung für eine Patientenliege als Komponente beispielsweise einen Riementrieb mit einem Motor aufweisen kann. Dabei werden als Mechanikeigenschaften vorliegend die Reibung und das Spiel betrachtet. Diese können zumindest teilweise abhängig von Betriebszustandsinformation sein, beispielsweise von einer aktuellen Position der Komponente bzw. der Antriebseinrichtung, einer Temperatur und insbesondere auch einem Gewicht des Patienten, welches beispielsweise über eine entsprechende Lasterfassungseinrichtung erfasst werden kann. Dabei muss nicht unbedingt eine konkrete Messung an dem Patiententisch vorgenommen werden, um die zu bewegende Last zu bestimmen, sondern es können beispielsweise auch Angaben zum Gewicht eines Patienten aus einer elektronischen Patientenakte oder dergleichen entnommen werden.

Selbstverständlich sind jedoch auch andere Konfiguration denkbar, in denen die Erfindung angewendet werden kann.

In einem Schritt S1 wird nach Beendigung der Fertigung des Patiententisches, jedoch vor Installation am Betriebsort, eine erste Ermittlung der die Mechanikeigenschaften beschreibenden Mechanikinformation vorgenommen, vorliegend durch eine Messung. Dabei werden konkrete Messungen von Reibung und Spiel vorgenommen und in Beziehung zu bei einer Messung des Motordrehmoments auftretenden Verläufen gesetzt, wobei das Spiel in Beziehung zu einem Zeitraum vor dem Anfahrtspeak gesetzt wird und der Reibwert in Beziehung zu den mittleren Wert des Motordrehmoments bei Konstantfahrt. Mittels einer so erhaltenen Mechanikinformation werden ursprüngliche Kompensationsparameter für die Steuereinrichtung des Patiententischs bestimmt, welche bei einer Bewegungsanforderung in einem Ermittlungsvorgang genutzt werden, um Steuerparameter für den Motor zu ermitteln, der dann die Bewegung gemäß der Bewegungsanforderung durchführt. Dabei wird aufgrund der Berücksichtigung der Mechanikeigenschaften eine bestimmte, gewollte Positioniergenauigkeit erreicht. Die Mechanikinformation wird im Schritt S1 ferner auch genutzt, um ein Simulationsmodell, welches hier den gesamten Patiententisch abbildet, bezüglich der Mechanikeigenschaften zu parametrieren. Dieses Simulationsmodell dient im weiteren Verlauf als digitaler Zwilling des Patiententischs und kann, ebenso wie ein Motorcontroller des Motors, einen Verlauf des Motordrehmoments als ein Simulationsergebnis generieren.

Der Patiententisch wird nun am Betriebsort installiert, insbesondere montiert, wonach in einem Schritt S2, vor Inbetriebnahme des Patiententisches, eine Überprüfungsmessung durchgeführt wird, beispielsweise bestimmte Fahrten entlang einer Achse durchgeführt werden, zu denen jeweils der Motordrehmomentverlauf als Messergebnis der Überprüfungsmessung ermittelt wird. Gleichzeitig dazu werden die entsprechenden Fahrten auch mittels des Simulationsmodells simuliert, welches als Simulationsergebnis der Überprüfungsmessung ebenso Motordrehmomentverläufe erzeugt.

Das Messergebnis der Überprüfungsmessung und das Simulationsergebnis des Simulationsmodells werden in einem Schritt S3 mittels mehrere Maßnahmenkriterien verglichen. Ein erstes Maßnahmenkriterium prüft dabei auf eine erste, kleinere Abweichung. Ist es erfüllt, ist es gemäß dem Schritt S4 ausreichend, die Kompensationsparameter sowie die Parametrisierung des Simulationsmodells entsprechend zu aktualisieren, vgl. Schritt S4. Ist jedoch ein zweites, auf eine größere Abweichung prüfendes Maßnahmenkriterium im Schritt S3 erfüllt, lässt dies auf einen Montagefehler bzw. allgemein Installationsfehler schließen, sodass eine entsprechende Nachricht ausgegeben wird und abgebrochen wird, um den Installationsfehler beheben zu können, vgl. Schritt S5.

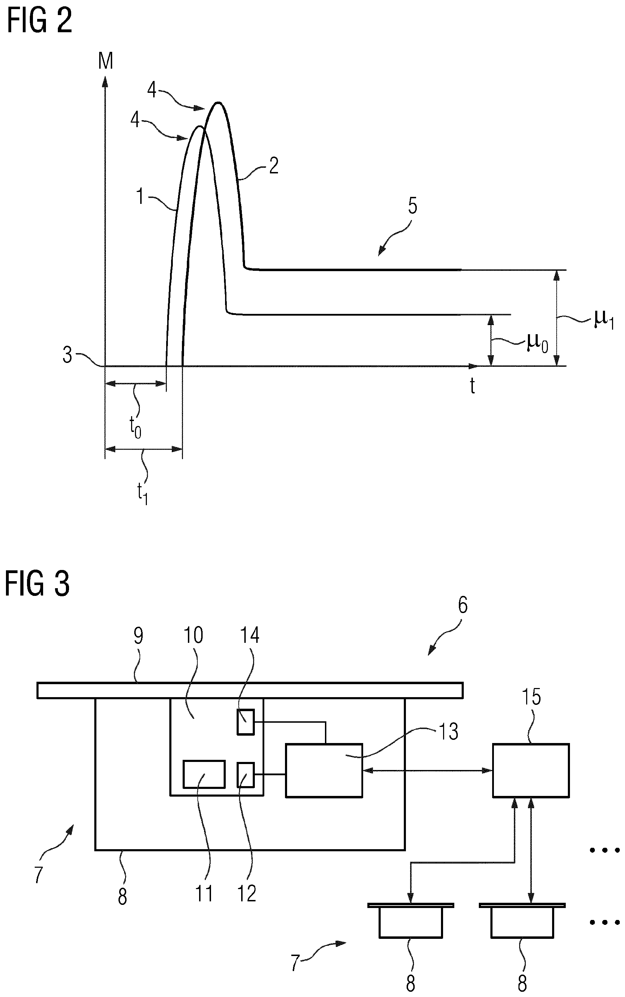

Fig. 2 zeigt in diesem Zusammenhang beispielhaft zwei aufgenommene Verläufe 1, 2 des Motordrehmoments M gegen die Zeit t. Ersichtlich existiert nach dem Startzeitpunkt 3 jeweils eine Zeitspanne t₀ für den Verlauf 1 bzw. t₁ für den Verlauf 2, in der noch kein Motordrehmoment aufgebaut wird, sodass diese Zeitspanne vor dem dann folgenden Anfahrtspeak 4 das Spiel in dem momentanen Betriebszustand, beschrieben durch die Betriebszustandsinformation, beschreibt. Nach dem Anfahrtspeak 4 erreichen beide Verläufe einen Konstantfahrtbereich 5, in dem jedoch hier unterschiedliche mittlere Motordrehmomente µ₀, µ₁ vorliegen, die ein Maß für die Reibung sind. Eine zum Abschluss des Bewegungsvorgangs folgende Bremsrampe ist vorliegend nicht gezeigt.

Werden die beiden Verläufe 1, 2 verglichen, stellt man fest, dass sich vom Verlauf 1 zum Verlauf 2 (unter Annahme vergleichbarer, insbesondere gleicher, Betriebszustände) das Spiel gemäß der Änderung von t₀ auf t₁ und die Reibung gemäß der Änderung von µ₀ auf µ₁ verändert hat.

Im Rahmen der hier beschriebenen Ausführungsbeispiele kann beispielsweise der Verlauf 1 ein Simulationsergebnis sein, der Verlauf 2 ein Messergebnis des entsprechenden simulierten Bewegungsvorgangs. Mithin ist aus den Verläufen 1 und 2 nicht nur ersichtlich, ob die Simulation gemäß dem digitalen Zwilling noch der Realität entspricht, sondern auch, in welchem Maß sich die Mechanikeigenschaften Spiel und Reibung - zumindest für den entsprechenden Betriebszustand - verändert haben.

Dies wird im Verfahren gemäß Fig. 1 ausgenutzt, indem jedes Mal, wenn eine Bewegungsanforderung einem Schritt S6 empfangen wird, nicht nur gemäß Schritt S7 unter Nutzung der aktuellen Kompensationsparameter im Ermittlungsvorgang eine Ansteuerung der Antriebseinrichtung, hier konkret des Motors, gemäß den aktuellen Kompensationsparametern erfolgt, wobei wiederum ein Messergebnis für das Motordrehmoment aufgenommen wird, sondern gleichzeitig der digitale Zwilling, also das Simulationsmodell, im Schritt S8 dieselbe Bewegung gemäß der Bewegungsanforderung durchführt und als Simulationsergebnis ebenso einen Verlauf 1, 2 des Motordrehmoments erhält. Dies dient der Überwachung, ob die Kompensationsparameter noch hinreichend genau sind, dass die gewollte Positioniergenauigkeit erreicht wird.

Daher wird in einem Schritt S9 ein Aktualisierungskriterium ausgewertet, welches die Abweichung zwischen dem Messergebnis und dem Simulationsergebnis, hier beispielsweise den Verläufen 1, 2, bewertet, beispielsweise, indem gegen Schwellwerte für t₁-t₀ und/oder µ₁-µ₀ geprüft. Liegt eine zu starke Abweichung zwischen dem Messergebnis und dem Simulationsergebnis vor, wird in einem Schritt S10 die Mechanikinformation aus dem Messergebnis neu ermittelt und es werden die Kompensationsparameter angepasst (Neukalibrierung) sowie das Simulationsmodell aktualisiert (Neuparametrierung). Damit sind der digitale Zwilling und die Steuereinrichtung wieder auf demselben Stand, und zwar derart, dass die gewollte Positioniergenauigkeit sicher erreicht wird. Auf diese Weise kann die Positioniergenauigkeit letztlich über die gesamte Lebenszeit des Patiententisches bzw. allgemein der Medizintechnikeinrichtung aufrechterhalten werden. Es sei noch angemerkt, dass dann, wenn die Mechanikeigenschaften bzw. konkret die Mechanikeigenschaftsinformation betriebszustandsabhängig betrachtet wird, beispielsweise abhängig von der Position der zu bewegenden Last, bei der Neuermittlung der Mechanikinformation noch weitere Messergebnisse gesammelt werden können, die sich auf verschiedene Betriebszustände beziehen und so eine Anpassung über den gesamten sinnvollen Wertebereich erlauben.

Ferner sei an dieser Stelle noch angemerkt, dass die Position in der Steuereinrichtung üblicherweise ohnehin mitgeführt wird, wobei die zu bewegende Last anhand eines Patientengewichts, welches durch Wiegen oder anderweitig, beispielsweise aus einer elektronischen Patientenakte oder sogar dem Motordrehmoment, bestimmt werden kann, ermittelt werden kann. Wird auch eine Temperatur berücksichtigt, kann diese gegebenenfalls auch durch den entsprechend ausgebildeten Motorcontroller bereitgestellt werden.

In einem Schritt S11 werden Protokolldaten für eine weitere Verwendung gespeichert. Diese Protokolldaten können beispielsweise das Messergebnis und das Simulationsergebnis für jeden durchgeführten Bewegungsvorgang sowie ermittelte Abweichungen umfassen, wobei es auch ausreichend sein kann, nur aktuelle Einstellungen (Mechanikeigenschaften und/oder Kompensationsparameter und/oder Simulationsparameter) und/oder Abweichungen als Protokolldaten abzuspeichern. In jedem Fall wird zweckmäßigerweise auch der Betriebszustand in Form der Betriebszustandsinformation protokolliert, ferner auch der durchgeführte Bewegungsvorgang, da beispielsweise durch Vergleich von kürzeren und längeren Bewegungsvorgängen ebenso Schlussfolgerungen bezogen werden können, beispielsweise bezüglich Beschädigungen, Verschleiß und Wartungsbedürftigkeit.

Diesbezüglich werden nämlich im Schritt S12 die Protokolldaten ausgewertet, um eine Verschleißinformation und eine die Notwendigkeit einer Wartung angebende Wartungsinformation zu ermitteln, die dann durch entsprechende Verschleißkriterien und/oder Wartungskriterien ausgewertet werden können, um beispielsweise eine Ausgabe zumindest eines Teils der Verschleißinformation und/oder der Wartungsinformation an einen Benutzer zu erzwingen, was in einem Schritt S13 geschehen kann. Beispielsweise können dann Schwellwerte herangezogen werden, die beispielsweise beschreiben, wie stark sich Reibung und/oder Spiel verändern dürfen, um auf eine Wartungsbedürftigkeit schließen zu können. Bezüglich der Verschleißinformation kann beispielsweise auch auf vorliegende Defekte, beispielsweise in Lagern oder dergleichen, geschlossen werden. Hierzu kann beispielsweise eine Früherkennung von Antriebseinrichtungsschäden, beispielsweise Getriebe- und/oder Lagerschäden, mittels Überwachung der Veränderung über den Bewegungsweg, hier Achs-Verfahrweg, durchgeführt werden.

Für die nächste Bewegungsanforderung wird wieder mit S6 fortgefahren.

Es sei angemerkt, dass das Simulationsmodell vorliegend auf einer medizintechnikeinrichtungsexternen Servereinrichtung, beispielsweise einer Cloud, abgelegt wird, wo auch die Protokolldaten gespeichert werden können. Das Ablegen in einer externen Servereinrichtung ist bereits bezüglich der Schritte S1 und S2 sinnvoll, da dann von überall auf das Simulationsmodell zugegriffen werden kann. Gleichzeitig ermöglicht es die Nutzung einer Servereinrichtung auch, Simulationsmodelle und Protokolldaten verschiedener Medizintechnikeinrichtung gemeinsam vorzuhalten, was es insbesondere gemäß Schritt S14 auch erlaubt, eine gemeinsame Auswertung über individuelle Medizintechnikeinrichtungen hinweg vorzunehmen. Hieraus können nicht nur beispielweise problematische Chargen detektiert werden bzw. allgemeine Informationen zu Lebensdauer, Verschleiß und Wartung gesammelt werden, sondern auch, wenn Betriebszustandsinformationen und Bewegungsvorgänge beschreibende Bewegungsdaten abgespeichert werden, auf die übliche Auslastung geschlossen werden. Alle diese Information erweisen sich als hochgradig nützlich bei der Entwicklung neuer Antriebseinrichtungen bzw. allgemein Medizintechnikeinrichtungen.

Fig. 3 zeigt eine schematische Prinzipskizze einer erfindungsgemäßen Medizintechnikanordnung 6. Diese umfasst vorliegend mehrere Medizintechnikeinrichtungen 7, vorliegend beispielhaft Patiententische 8, von denen einer genauer gezeigt ist. Der Patiententisch 8 weist als bewegbare Komponente eine Patientenliege 9 auf, die beispielsweise über entsprechende Antriebseinrichtungen 10 entlang der Vertikalachse, der Längsachse und der Querachse bewegbar sein kann. Dabei sein im Übrigen im Hinblick auf das Verfahren noch angemerkt, dass selbstverständlich mehrere Antriebseinrichtungen 10 pro Medizintechnikeinrichtung 7 in dem Verfahren berücksichtigt werden können, insbesondere dann, wenn sich das Simulationsmodell auf alle Antriebseinrichtungen 10, insbesondere die gesamte Medizintechnikeinrichtung 7, bezieht.

Vorliegend ist der Übersichtlichkeit halber nur eine Antriebseinrichtung 10 mit ihren Komponenten schematisch angedeutet. Die Antriebseinrichtung 10, welche beispielsweise als ein Riementrieb ausgebildet sein kann, umfasst einen Motor 11, der über einen Motorcontroller 12 mittels einer Steuereinrichtung 13 des Patiententisches 8 angesteuert wird. Der Motorcontroller ist ein Mehrzweck-Elektronikbauteil, welches an definierten Schnittstellen ohnehin bereits Werte für das Motordrehmoment und die Temperatur bereitstellt, sodass das Messergebnis auf unkomplizierte Art und Weise der Steuereinrichtung 13 bereitgestellt werden kann. Die Steuereinrichtung 13 kann auch Betriebszustandsinformation über eine zu bewegende Last, beispielsweise ein Patientengewicht, mittels einer Lasterfassungseinrichtung 14 erhalten.

Die Steuereinrichtungen 13 der Patiententische 8 bilden gemeinsam mit der zentralen Servereinrichtung 15, auf der die Simulationsmodelle und die Protokolldaten abgelegt sein können und mit der die Steuereinrichtungen 13 kommunizieren, eine Steueranordnung, welche zur Durchführung des erfindungsgemäßen Verfahrens ausgebildet ist. Insbesondere führt die Steuereinrichtung 13 den Ermittlungsvorgang und die konkrete Ansteuerung durch, während die Simulation und die Auswertung der diversen Kriterien in der Servereinrichtung 15, welche ebenso ein Prozessor und ein Speichermittel aufweist, erfolgen können.

## Patentansprüche

1. Verfahren zum Betrieb einer Medizintechnikeinrichtung (7), nämlich einer medizinischen Bildgebungseinrichtung, wobei die Medizintechnikeinrichtung (7) wenigstens eine mittels einer Antriebseinrichtung (10), die durch eine Steuereinrichtung (13) ansteuerbar ist, positionierbare Komponente aufweist,
wobei bei Empfang einer Bewegungsanforderung für die Komponente mittels eines Ermittlungsvorgangs Steuerparameter für die Antriebseinrichtung (10) durch die Steuereinrichtung (13) ermittelt werden und die Antriebseinrichtung (10) gemäß der Steuerparameter zur Umsetzung der Bewegungsanforderung angesteuert wird,
wobei in den Ermittlungsvorgang wenigstens ein Kompensationsparameter eingeht, der aus einer Mechanikeigenschaftsinformation, die wenigstens eine auf die Positionierungsgenauigkeit der Antriebseinrichtung (10) bezogene Mechanikeigenschaft beschreibt, bestimmt wird,
wobei zur Überwachung und/oder Ermittlung und/oder Anpassung des wenigstens einen Kompensationsparameters ein Simulationsergebnis eines wenigstens den Betrieb der Antriebseinrichtung (10) abbildenden Simulationsmodells verwendet wird, das mittels wenigstens eines aufgrund einer Ermittlung der Mechanikeigenschaftsinformation zu einem Simulationsmodellparametrierungszeitpunkt bestimmten Simulationsparameter parametriert ist,
wobei als ein erster Simulationsmodellparametrierungszeitpunkt eine Beendigung der Herstellung verwendet wird, wobei dann auch eine Kalibrierung der Steuereinrichtung (13) bezüglich der Kompensationsparameter erfolgt,
wobei bei Inbetriebnahme nach der Installation eine Überprüfungsmessung durchgeführt wird, bei der das Simulationsergebnis mit einem Messergebnis der Überprüfungsmessung verglichen wird,
wobei bei Erfüllung eines ersten, eine erste, kleinere Abweichung zwischen dem Simulationsergebnis und der Überprüfungsmessung anzeigenden Maßnahmenkriteriums eine Neukalibrierung der Steuereinrichtung (13) und eine Neuparametrierung des Modells durchgeführt wird und
bei Erfüllung eines zweiten, eine zweite, größere Abweichung zwischen dem Simulationsergebnis und der Überprüfungsmessung anzeigenden Maßnahmenkriteriums eine einen Fehler bei der Installation anzeigende Warnmeldung ausgegeben wird und
wobei die o. **g.** Verfahrensschritte automatisiert durch die Steuereinrichtung (13) ausgeführt werden.

2. Verfahren nach Anspruch 1, **dadurch gekennzeichnet, dass** als Mechanikeigenschaft eine Reibung und/oder ein Spiel und/oder eine Steifigkeit der Antriebseinrichtung (10) verwendet wird und/oder dass wenigstens eine Mechanikeigenschaftsinformation in Abhängigkeit von wenigstens einer den Betriebszustand der Antriebseinrichtung (10) betreffenden Betriebszustandsinformation ermittelt wird.

3. Verfahren nach Anspruch 2, **dadurch gekennzeichnet, dass** als Betriebszustandsinformation eine aktuelle Position der Komponente und/oder eine zu bewegende Last verwendet wird und/oder die Betriebszustandsinformation auch in den Ermittlungsvorgang und/oder die Simulation eingeht.

4. Verfahren nach einem der vorangehenden Ansprüche, **dadurch gekennzeichnet, dass** zur Überwachung und/oder Anpassung der Kompensationsparameter ein während der Ausführung einer Bewegungsanforderung aufgenommenes Messergebnis mit einem die Ausführung der Bewegungsanforderung in dem Simulationsmodell beschreibenden Simulationsergebnis verglichen wird.

5. Verfahren nach Anspruch 4, **dadurch gekennzeichnet, dass** ein Verlauf (1, 2) eines Motorstroms und/oder eines Motordrehmoments eines Motors (11) der Antriebseinrichtung (10) vermessen und simuliert werden.

6. Verfahren nach Anspruch 4 oder 5, **dadurch gekennzeichnet, dass** bei einer ein Aktualisierungskriterium erfüllenden Abweichung zwischen dem Messergebnis und dem Simulationsergebnis durch Neuermittlung der Mechanikeigenschaftsinformation die Kompensationsparameter und die Simulationsparameter zur Korrektur der Abweichung angepasst werden.

7. Verfahren nach Anspruch 6, **dadurch gekennzeichnet, dass** die Mechanikeigenschaftsinformation unter Verwendung des Messergebnisses neu ermittelt wird.

8. Verfahren nach einem der Ansprüche 4 bis 7, **dadurch gekennzeichnet, dass** der Vergleich für jede Bewegungsanforderung durchgeführt wird.

9. Verfahren nach einem der Ansprüche 4 bis 8, **dadurch gekennzeichnet, dass** jeder Vergleich und/oder jede Anpassung protokolliert werden, wobei durch Auswertung der Protokolldaten wenigstens eine Verschleißinformation und/oder eine die Notwendigkeit einer Wartung angebende Wartungsinformation ermittelt werden.

10. Verfahren nach Anspruch 9, **dadurch gekennzeichnet, dass** die Protokollierung gemeinsam mit einer zugeordneten Betriebszustandsinformation erfolgt und/oder die Protokolldaten zur Ermittlung einer bei der Entwicklung neuer Antriebseinrichtungen (10) verwendbaren Entwicklungsinformation ausgewertet werden.

11. Verfahren nach einem der vorangehenden Ansprüche, **dadurch gekennzeichnet, dass** das Simulationsmodell auf einer zur Medizintechnikeinrichtung (7) externen, mit dieser kommunizierenden Servereinrichtung (15) abgespeichert wird.

12. Medizintechnikanordnung (6), umfassend eine Steueranordnung und wenigstens eine Medizintechnikeinrichtung (7), wobei die Medizintechnikeinrichtung (7) wenigstens eine mittels einer Antriebseinrichtung (10), die durch eine Steuereinrichtung (13) als Teil der Steueranordnung ansteuerbar ist, positionierbare Komponente aufweist, **dadurch gekennzeichnet, dass** die Steueranordnung zur Durchführung eines Verfahrens nach einem der vorangehenden Ansprüche ausgebildet ist.

13. Medizintechnikanordnung (6) nach Anspruch 12, **dadurch gekennzeichnet, dass** sie mehrere Medizintechnikeinrichtungen (7) und eine Servereinrichtung (15) zum Vorhalten der Simulationsmodelle aller Medizintechnikeinrichtungen (7) aufweist.

## Claims

1. Method for operating a medical technology facility (7), namely a medical imaging facility, wherein the medical technology facility (7) has at least one component which can be positioned by means of a drive facility (10) which can be controlled by a control facility (13),
wherein, upon receipt of a movement request for the component, control parameters for the drive facility (10) are determined by the control facility (13) by means of a determination process and the drive facility (10) is controlled to implement the movement request in accordance with the control parameters,
wherein at least one compensation parameter is included in the determination process, which parameter is ascertained from an item of information on mechanical properties, which describes at least one mechanical property relating to the positioning accuracy of the drive facility (10),
wherein a simulation result from a simulation model which maps at least the operation of the drive facility (10) is used for monitoring and/or determining and/or adjusting the at least one compensation parameter, which result is parameterised by means of at least one ascertained simulation parameter based on a determination of the mechanical property information on a simulation model parameterisation time instant,
wherein production completion is used as a first simulation model parameterisation time instant, wherein a calibration of the control facility (13) then also takes place relative to the compensation parameters,
wherein during commissioning, after installation, a test measurement is performed, in which the simulation result is compared with a measurement result of the test measurement,
wherein on fulfilment of a first measurement criterion indicating a first, smaller deviation between the simulation result and the test measurement, a recalibration of the control facility (13) and a reparameterisation of the model is performed and
on fulfilment of a second measurement criterion indicating a second, larger deviation between the simulation result and the test measurement, a warning notification which indicates an installation fault is output and
wherein the above-mentioned method steps are automatically performed by the control facility (13).

2. Method according to claim 1, **characterised in that** friction and/or play and/or rigidity of the drive facility (10) are used as a mechanical property and/or that at least one item of information on mechanical property is determined as a function of at least one item of operation status information relating to the operation status of the drive facility (10).

3. Method according to claim 2, **characterised in that** a current position of the component and/or load to be moved is used as operation status information and/or the operation status information is also included in the determination process and/or the simulation.

4. Method according to one of the preceding claims, **characterised in that**, for monitoring and/or adjusting the compensation parameters, a measurement result recorded during the performance of a movement request is compared with a simulation result describing the performance of the movement request in the simulation model.

5. Method according to claim 4, **characterised in that** a curve (1, 2) of a motor current and/or a motor torque of a motor (11) of the drive facility (10) is measured and simulated.

6. Method according to claim 4 or 5, **characterised in that**, where a deviation between the measurement result and the simulation result occurs which meets an updating criterion, the compensation parameter and the simulation parameter can be adjusted to correct the deviation by redetermining the item of information on mechanical property.

7. Method according to claim 6, **characterised in that** the item of information on mechanical property is redetermined using the measurement result.

8. Method according to one of claims 4 to 7, **characterised in that** the comparison is performed for each movement request.

9. Method according to one of claims 4 to 8, **characterised in that** each comparison and/or adjustment is logged, wherein at least one item of information relating to wear and/or one item of maintenance information indicating the need for maintenance is determined by evaluating the log data.

10. Method according to claim 9, **characterised in that** logging takes place together with an assigned item of operation status information and/or the log data is evaluated to determine an item of development information which can be used for developing new drive facilities (10).

11. Method according to one of the preceding claims, **characterised in that** the simulation model is stored on a server facility (15) which is external to the medical technical facility (7) and which communicates with the medical technical facility.

12. Medical technical arrangement (6), comprising a control arrangement and at least one medical technical facility (7), wherein the medical technical facility (7) has at least one component which can be positioned by means of a drive facility (10) which can be controlled by a control facility (13) as part of the control arrangement, **characterised in that** the control arrangement is designed to carry out a method according to one of the preceding claims.

13. Medical technical arrangement (6) according to claim 12, **characterised in that** it has a number of medical technical facilities (7) and a server facility (15) for providing the simulation models of all of the medical technical facilities (7).

## Revendications

1. Procédé pour faire fonctionner un dispositif (7) de la technique médicale, à savoir un dispositif d'imagerie médicale,
dans lequel le dispositif (7) de la technique médicale a au moins un composant, qui peut être mis en position au moyen d'un dispositif (10) d'entraînement, lequel peut être commandé par un dispositif (13) de commande,
dans lequel, à la réception d'une demande de déplacement du composant, on détermine par le dispositif (13) de commande au moyen d'une opération de détermination des paramètres de commande du dispositif (10) d'entraînement et on commande le dispositif (10) d'entraînement suivant les paramètres de commande pour la mise en œuvre de la demande de déplacement, dans lequel dans l'opération de détermination entre au moins un paramètre de compensation, que l'on détermine à partir d'une information de propriété mécanique, qui décrit au moins une propriété mécanique rapportée à la précision de positionnement du dispositif (10) d'entraînement,
dans lequel pour le contrôle et/ou la détermination et/ou l'adaptation du au moins un paramètre de compensation, on utilise un résultat de simulation d'un modèle de simulation, qui reproduit un résultat de simulation d'au moins le fonctionnement du dispositif (10) d'entraînement et qui est paramétré au moyen d'un paramètre de simulation déterminé sur la base d'une détermination de l'information de propriété mécanique à un instant de paramétrisation du modèle de simulation,
dans lequel
on utilise comme premier instant de paramétrage du modèle de simulation une fin de la production, dans lequel il s'effectue également alors un étalonnage du dispositif (13) de commande en ce qui concerne le paramètre de compensation, dans lequel
lors de la mise en service après l'installation, on effectue une mesure de contrôle, dans laquelle on compare le résultat de simulation à un résultat de la mesure de contrôle,
dans lequel, s'il est satisfait un premier critère de prise de mesure indiquant un premier écart assez petit entre le résultat de simulation et la mesure de contrôle, on effectue un nouvel étalonnage du dispositif (13) de commande et un nouveau paramétrage du modèle et
s'il est satisfait, un deuxième critère de prise de mesure indiquant un deuxième écart assez grand entre le résultat de simulation et la mesure de contrôle, on émet un message d'alerte indiquant un défaut dans l'installation et
dans lequel on effectue d'une manière automatisée par le dispositif (13) de commande les stades de procédé mentionnés ci-dessus.

2. Procédé suivant la revendication 1, **caractérisé en ce que** l'on utilise comme propriété mécanique un frottement et/ou un jeu et/ou une rigidité du dispositif (10) d'entraînement et/ou **en ce que** l'on détermine au moins une information de propriété mécanique en fonction d'au moins une information d'état de fonctionnement concernant l'état de fonctionnement du dispositif (10) d'entraînement.

3. Procédé suivant la revendication 2, **caractérisé en ce que** l'on utilise comme information d'état de fonctionnement une position en cours du composant et/ou d'une charge à déplacer et/ou l'information d'état de fonctionnement entre également dans l'opération de détermination et/ou la simulation.

4. Procédé suivant l'une des revendications précédentes, **caractérisé en ce que** pour le contrôle et/ou l'adaptation du paramètre de compensation, on compare un résultat de mesure enregistré pendant l'exécution d'une demande de mouvement à un résultat de simulation décrivant l'exécution de la demande de mouvement dans le modèle de simulation.

5. Procédé suivant la revendication 4, **caractérisé en ce que** l'on mesure et on simule une courbe (1, 2) d'un courant de moteur et/ou d'un couple d'un moteur (11) du dispositif (10) d'entraînement.

6. Procédé suivant la revendication 4 ou 5, **caractérisé en ce que**, s'il y a un écart satisfaisant un critère de mise à jour entre le résultat de mesure et le résultat de simulation, on adapte pour la correction de l'écart, par nouvelle détermination de l'information de propriété mécanique, le paramètre de compensation et le paramètre de simulation.

7. Procédé suivant la revendication 6, **caractérisé en ce que** l'on détermine à nouveau l'information de propriété mécanique en utilisant le résultat de mesure.

8. Procédé suivant l'une des revendications 4 à 7, **caractérisé en ce que** l'on effectue la comparaison pour chaque demande de mouvement.

9. Procédé suivant l'une des revendications 4 à 8, **caractérisé en ce que** l'on dresse un compte-rendu de chaque comparaison et/ou de chaque adaptation, dans lequel par l'évaluation des données du compte-rendu on détermine au moins une information d'usure et/ou une information d'entretien indiquant la nécessité d'un entretien.

10. Procédé suivant la revendication 9, **caractérisé en ce que** le compte-rendu a lieu conjointement avec une information d'état de fonctionnement associée et/ou on évalue les données du compte-rendu pour la détermination d'une information de développement pouvant être utilisée lors du développement de nouveaux dispositifs (10) d'entraînement.

11. Procédé suivant l'une des revendications précédentes, **caractérisé en ce que** l'on met le modèle de simulation en mémoire sur un dispositif (15) serveur extérieur au dispositif (7) de la technique médicale et communiquant avec celui-ci.

12. Agencement (6) de la technique médicale, comprenant un agencement de commande et au moins un dispositif (7) de la technique médicale, dans lequel le dispositif (7) de la technique médicale a au moins un composant pouvant être mis en position au moyen d'un dispositif (10) d'entraînement, lequel peut être commandé par un dispositif (13) de commande comme partie de l'agencement de commande, **caractérisé en ce que** l'agencement de commande est constitué pour l'exécution d'un procédé suivant l'une des revendications précédentes.

13. Agencement (6) de la technique médicale suivant la revendication 12, **caractérisé en ce qu'**il a plusieurs dispositifs (7) de la technique médicale et un dispositif (15) serveur pour conserver les modèles de simulation de tous les dispositifs (7) de la technique médicale.
